# EUROPEAN PATENT APPLICATION

(11) **EP 3 159 255 A1**
(43) Date of publication of application: **26.04.2017**
(21) Application number: 14894926.6
(22) Date of filing: 04.11.2014
(51) Int. Cl.: B63B 22/00, B63B 21/20, B63B 22/04

(54) **OCEAN DATA MEASUREMENT SYSTEM**

(30) Priority: 17.06.2014 JP 2014123957
(71) Applicant: IHI Corporation, Tokyo 135-8710 (JP)
(72) Inventor: ICHIKAWA, Masaaki, Tokyo 135-8710 (JP)
(74) Representative: MERH-IP Matias Erny Reichl Hoffmann Patentanwälte PartG mbB
(86) International application number: PCT/JP2014/079210
(87) International publication number: WO 2015/194062

(57) **Abstract**

A system includes: an anchor 3 placed on a sea bottom 2; a submersible mooring cable 4 having a first end connected to the anchor 3; an intermediate buoy 5 connected to a second end of the submersible mooring cable 4 and floating in the sea; an intermediate mooring cable 6 having a first end connected to the intermediate buoy 5; an observation buoy 8 connected to a second end of the intermediate mooring cable 6, and measuring ocean data of an upper layer from a sea surface 7 to the intermediate buoy 5 while floating and sinking between the intermediate buoy 5 and the sea surface 7; and a lower layer-observation unit 9 placed along the submersible mooring cable 4, and measuring ocean data of a lower layer in a location deeper than the intermediate buoy 5. Ocean data of a wide area between the sea surface and the sea bottom can be acquired easily.

## Description

### Technical Field

The present invention relates to an ocean data measurement system, and particularly relate to an ocean data measurement system capable of acquiring ocean data over a wide area, between the sea surface and the sea bottom.

### Background Art

It is known that an ocean data measurement system moored to a fixed point to measure ocean data such as water temperature and salinity concentration (conductivity) includes: an anchor placed on the sea bottom; a submersible mooring cable having a first end connected to the anchor; an intermediate buoy connected to a second end of the submersible mooring cable and floating in the sea; an intermediate mooring cable having a first end connected to the intermediate buoy; and an observation buoy connected to a second end of the intermediate mooring cable, and measuring ocean data from the sea surface to the intermediate buoy while floating and sinking between the intermediate buoy and the sea surface (see Patent Document 1, for example).

In the ocean data measurement system described in Patent Document 1, the observation buoy generally has a storage unit for storing measured ocean data. Ocean data is measured by floating and sinking the observation buoy between the sea surface and the intermediate buoy, and the ocean data is stored in the storage unit. Then, ocean data of depths between the sea surface and the intermediate buoy is acquired, by lifting the observation buoy up to the sea surface to stick an antenna provided in the observation buoy out from the sea surface, and transmitting the ocean data stored in the storage unit to a ground base or an observation ship, directly or through a satellite, for example.

### Related Art Document

### Patent Document

Patent Document 1: Japanese Patent Laid-Open No. 2013-103678

### Summary of the Invention

### Problems to be solved by the Invention

The above-mentioned ocean data measurement system measures ocean data, by floating and sinking the observation buoy between the sea surface and the intermediate buoy. Hence, although ocean data of an upper layer (area from the sea surface to the intermediate buoy) can be acquired, ocean data of locations deeper than the intermediate buoy (e.g. 300 m or deeper) cannot be measured.

Also, to sink the observation buoy deeper than the intermediate buoy, pressure resistance and floating-and-sinking performance of the observation buoy need to be improved, which inevitably increases cost. For example, there is a need to thicken the pressure-resistant shell of the observation buoy, and to improve the transmission capacity of a pump that feeds oil into a buoyancy bag for floating and sinking.

The present invention has been made in view of the problems described above, and aims to provide an ocean data measurement system that can easily acquire ocean data of a wider area, between the sea surface and the sea bottom.

### Means for Solving the Problems

The present invention is an ocean data measurement system including: an anchor placed on a sea bottom; a submersible mooring cable having a first end connected to the anchor; an intermediate buoy connected to a second end of the submersible mooring cable and floating in the sea; an intermediate mooring cable having a first end connected to the intermediate buoy; an observation buoy connected to a second end of the intermediate mooring cable, and measuring ocean data of an upper layer from a sea surface to the intermediate buoy while floating and sinking between the intermediate buoy and the sea surface; and a lower layer-observation unit placed along the submersible mooring cable, and measuring ocean data of a lower layer in a location deeper than the intermediate buoy.

### Advantageous Effects of the Invention

According to the ocean data measurement system of the present invention, ocean data of the upper layer from the sea surface to the intermediate buoy can be measured by the observation buoy, while ocean data of the lower layer in locations deeper than the intermediate buoy can be measured by the lower layer-observation unit. Hence, ocean data can be acquired for a wider area than in conventional systems. Additionally, since the observation buoy need not be sunk deeper than the intermediate buoy, the ocean data measurement system acquiring ocean data of a wide area can be constructed at low cost.

### Brief Description of the Drawings

FIG. 1 is an explanatory schematic drawing of an entire ocean data measurement system according to an embodiment of the present invention.
FIG. 2 is a partial enlargement of the ocean data measurement system shown in FIG. 1.
FIG. 3A is a diagram showing a first modification of the lower layer-observation unit shown in FIG. 1.
FIG. 3B is a diagram showing a second modification of the lower layer-observation unit shown in FIG. 1.
FIG. 3C is a diagram showing a third modification of the lower layer-observation unit shown in FIG. 1.

### Mode for Carrying out the Invention

Hereinafter, an embodiment of the present invention will be described in detail, with reference to the accompanying drawings. Dimensions, materials, and specific numeric values, for example, indicated in the embodiment are only examples for facilitating understanding of the invention, and do not limit the present invention unless stated otherwise. Note that in the specification and drawings, components having substantially the same functions and configurations are assigned with the same reference signs to omit overlapping descriptions, and components not directly related to the present invention are omitted from the drawings.

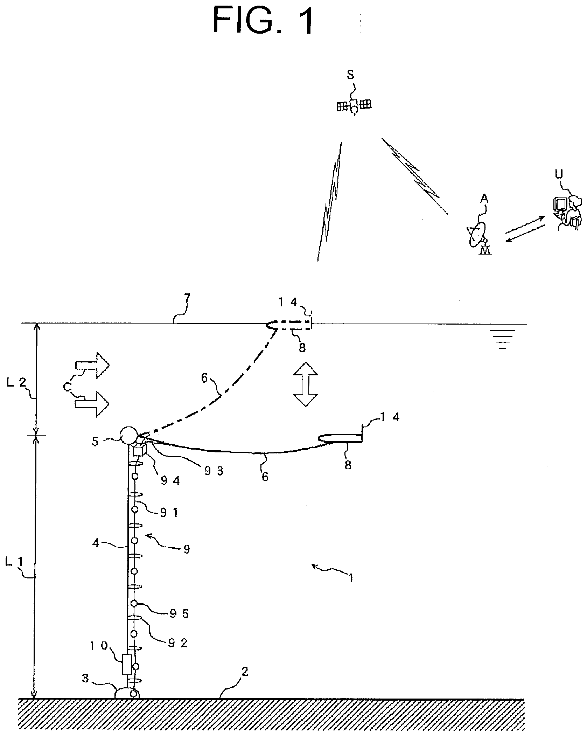

As shown in FIG. 1, an ocean data measurement system 1 according to an embodiment of the present invention includes: an anchor 3 placed on a sea bottom 2; a submersible mooring cable 4 having a first end connected to the anchor 3; an intermediate buoy 5 connected to a second end of the submersible mooring cable 4 and floating in the sea; an intermediate mooring cable 6 having a first end connected to the intermediate buoy 5; an observation buoy 8 connected to a second end of the intermediate mooring cable 6, and measuring ocean data of an upper layer from a sea surface 7 to the intermediate buoy 5 while floating and sinking between the intermediate buoy 5 and the sea surface 7; and a lower layer-observation unit 9 placed along the submersible mooring cable 4, and measuring ocean data of a lower layer in locations deeper than the intermediate buoy 5.

The anchor 3 is placed on the sea bottom 2 of an observation area for which ocean data including water temperature, hydraulic pressure, and salinity concentration (conductivity) needs to be measured. The anchor 3 moors the intermediate buoy 5 in the sea in the observation area, through the submersible mooring cable 4. The observation buoy 8 is connected to the intermediate buoy 5 through the intermediate mooring cable 6. The observation buoy 8 is placed within the observation area by the anchor 3, the submersible mooring cable 4, the intermediate buoy 5, and the intermediate mooring cable 6.

As the anchor 3, a weight is used which is heavy enough not to shift on the sea bottom 2, when the intermediate buoy 5 and the observation buoy 8 receive force of an ocean current C and pull the submersible mooring cable 4. Note that the anchor 3 may be a pile or other member fixed to the sea bottom 2. Although the depth of the sea bottom 2 where the anchor 3 is placed is about 1000 m, for example, in the embodiment, the invention is not limited to this depth.

The submersible mooring cable 4 has a first end connected to the anchor 3 and a second end connected to the intermediate buoy 5, and holds the intermediate buoy 5 to a fixed point in the sea. The submersible mooring cable 4 is formed of a rope in which multiple strings such as resin are twisted together, for example. The length of the submersible mooring cable 4 is set appropriately, depending on the depth at which the intermediate buoy 5 needs to be placed. For example, the length of the submersible mooring cable 4 is set such that a distance L1 from the sea bottom 2 to the intermediate buoy 5 is about 700 m. In this case, a distance L2 from the intermediate buoy 5 to the sea surface 7 is about 300 m. In the embodiment, the area of the distance L1 corresponds to a lower layer, and the area of the distance L2 corresponds to an upper layer.

An acoustic release device 10 may be provided in a lower part of the submersible mooring cable 4. The acoustic release device 10 has a function of releasing the submersible mooring cable 4, upon receipt of an acoustic wave having a predetermined frequency. When ocean data measurement by the ocean data measurement system 1 is completed, an observation ship on the sea surface 7 may transmit an acoustic wave having a predetermined frequency, toward the acoustic release device 10 at the sea bottom 2. This allows the acoustic release device 10 to release the submersible mooring cable 4. With this process, the observation ship can collect system components such as the submersible mooring cable 4, the intermediate mooring cable 6, and the observation buoy 8, which are lifted up to the sea surface 7 by buoyancy of the intermediate buoy 5.

The intermediate buoy 5 is moored to a fixed point in the sea by being connected to the anchor 3 through the submersible mooring cable 4, and forms a starting point of floating and sinking of the observation buoy 8, which is connected through the intermediate mooring cable 6.
Buoyancy of the intermediate buoy 5 is set such that the intermediate buoy 5 is not carried too far away to the downstream side by the ocean current C, and that the anchor 3 does not shift on the sea bottom 2.

In the embodiment, since the depth (distance L2) of the intermediate buoy 5 is set to about 300 m, sunlight is less likely to reach the intermediate buoy 5 and the submersible mooring cable 4, so that attachment of marine organisms to the intermediate buoy 5 and the submersible mooring cable 4 can be suppressed. Accordingly, it is possible to continuously observe ocean data over an extended period (about half to one year). Also, since fishing nets are generally used at the depth of about 200 m at the deepest, interference of the intermediate buoy 5 and submersible mooring cable 4 with fishing nets can also be avoided.

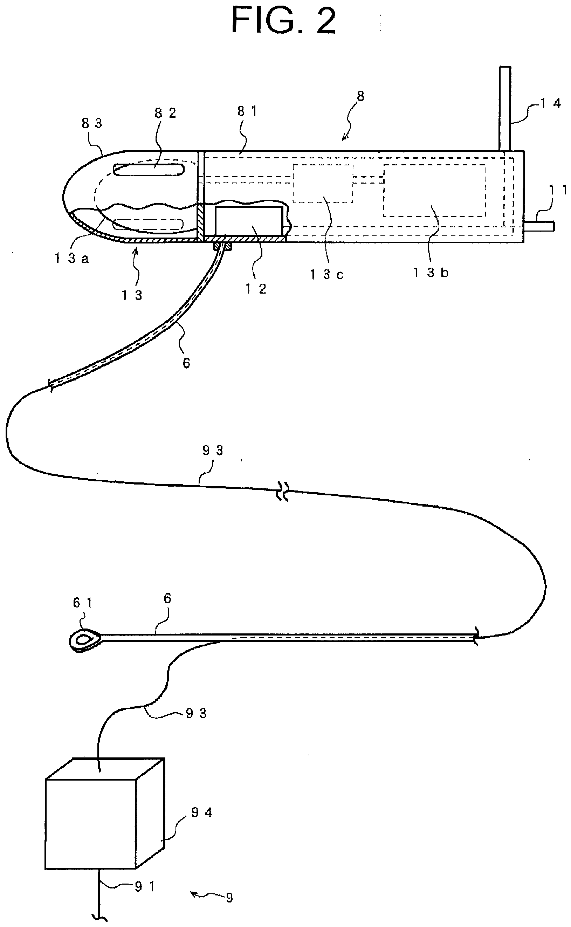

The intermediate mooring cable 6 has a first end connected to the intermediate buoy 5 and a second end connected to the observation buoy 8, and moors the observation buoy 8 to the intermediate buoy 5. The intermediate mooring cable 6 is formed of a rope in which multiple strings such as resin are twisted together, for example. As shown in FIG. 2, the intermediate mooring cable 6 has, on one end thereof, a ring portion 61 connected to the intermediate buoy 5.

The length of the intermediate mooring cable 6 is set such that the observation buoy 8 can float up to the sea surface 7 from the depth of the intermediate buoy 5, is set at least equal to or longer than the distance L2 (e.g. 300 m) from the sea surface 7 to the intermediate buoy 5, and is set to a predetermined length (e.g. 600 m) depending on the intensity of the ocean current C. Note that the specific gravity of the intermediate mooring cable 6 may be set equivalent to that of sea water, so that self-weight of the intermediate mooring cable 6 does not sink the observation buoy 8.

The observation buoy 8 is connected to the intermediate buoy 5 through the intermediate mooring cable 6, and measures ocean data of the upper layer from the sea surface 7 to the intermediate buoy 5 by floating and sinking between the intermediate buoy 5 and the sea surface 7. As shown in FIG. 2, the observation buoy 8 is mainly configured of a hollow cylindrical pressure-resistant shell 81, and a dome-like cover 83, which is attached to the tip end of the pressure-resistant shell 81 and has a through-hole 82 formed therein, for example. The observation buoy 8 has a measuring portion 11 for measuring ocean data, a storage unit 12 (e.g. memory) for storing measured ocean data, and a specific gravity-adjustment mechanism 13 for adjusting the depth of the observation buoy 8.

The measuring portion 11is at least one of a CTD sensor (Conductivity, Temperature, and Depth) for acquiring conductivity (salinity concentration) and other data; a pressure sensor; a magnetometric sensor; and a radiation meter, for example. The types and number of sensors are appropriately selected, according to the type of ocean data that needs to be acquired in the observation area. Although the measuring portion 11 of the embodiment is provided at the tail of the pressure-resistant shell 81, it may be provided in an upper or a lower part of the pressure-resistant shell 81.

The storage unit 12 is configured of a semiconductor memory, a hard disk, or other parts accommodated in the pressure-resistant shell 81, and stores ocean data of the upper layer measured by the measuring portion 11. Note that as will be mentioned later, the storage unit 12 is capable of storing not only ocean data of the upper layer, but also ocean data of the lower layer in locations deeper than the intermediate buoy 5, which is measured by the lower layer-observation unit 9.

That is, the observation buoy 8 has the storage unit 12 that can store upper layer-ocean data and lower layer-ocean data. The storage unit 12 configured in this manner can collectively store ocean data of a wide area from the sea surface 7 to the sea bottom 2 in a concentrated manner, for example.

The specific gravity-adjustment mechanism 13 has a buoyancy bag 13a accommodated in the cover 83, and an oil pump 13c for injecting or discharging oil inside an oil tank 13b into or from the buoyancy bag 13a, for example. The oil tank 13b and oil pump 13c are accommodated in the pressure-resistant shell 81. While the inside of the pressure-resistant shell 81 is made watertight to prevent entry of seawater, the inside of the cover 83 is formed to allow entry of seawater through the through-hole 82.

Accordingly, injecting oil into the buoyancy bag 13a and inflating the buoyancy bag 13a increases the apparent volume of the observation buoy 8, and decreases the specific gravity of the observation buoy 8. Hence, the observation buoy 8 is lifted up. Meanwhile, discharging oil from the buoyancy bag 13a and deflating the buoyancy bag 13a decreases the apparent volume of the observation buoy 8, and increases the specific gravity of the observation buoy 8. Hence, the observation buoy 8 is sunk.

During normal operation when not measuring ocean data, the specific gravity-adjustment mechanism 13 sinks the observation buoy 8 below the sea surface 7, and holds it at the depth (including area within plus and minus several tens of meters) of the intermediate buoy 5. This process can keep ships such as fishing boats from colliding with the observation buoy 8. Also, since the depth (distance L2) of the intermediate buoy 5 is set deeper (about 300 m) than the depth (about 0 m to 200 m) at which fishing nets are used, the observation buoy 8 held at the depth of the intermediate buoy 5 during normal operation can also be kept from interfering with fishing nets.

Also, since the depth of the intermediate buoy 5 is thus set to a depth (about 300 m) where sunlight is less likely to come through, marine organisms (crustaceans such as a barnacle, shells, and seaweeds) are less likely to attach to the observation buoy 8 and the intermediate mooring cable 6. Accordingly, it is possible to continuously observe ocean data over an extended period (about half to one year).

In addition, the observation buoy 8 has an antenna 14 for transmitting ocean data stored in the storage unit 12 to a user U in a ground base or on an observation ship, directly or through devices such as a satellite S and a parabolic antenna A. When transmitting ocean data in the storage unit 12 to the user U, the observation buoy 8 may be lifted up to the sea surface 7 as indicated by a dash-dot line in FIG. 1, and the antenna 14 on the observation buoy 8 sticks out from the sea surface 7.

Moreover, in the ocean data measurement system 1 according to the embodiment, a lower layer-observation unit 9 for measuring ocean data of the lower layer from the intermediate buoy 5 to the sea bottom 2 is placed along the submersible mooring cable 4, as shown in FIG. 1. The lower layer-observation unit 9 includes a sensor cable 91 placed along the submersible mooring cable 4, a restriction unit 92 for keeping the sensor cable 91 from separating from the submersible mooring cable 4, and a signal cable 93 for transmitting measured lower layer-ocean data to the observation buoy 8, for example. Also, the lower layer-observation unit 9 may have an electric watertight container 94 placed between the sensor cable 91 and the signal cable 93.

The sensor cable 91 has multiple sensors 95 spaced apart in the longitudinal direction of the submersible mooring cable 4 as shown in FIG. 1, for example. The sensor 95is at least one of a CTD sensor, a pressure sensor, a magnetometric sensor, and a radiation meter, for example. The sensor cable 91 may be an electric cable or an optical fiber cable, as long as it can transmit ocean data acquired by the sensors 95.

The sensor cable 91 has a first end connected to the electric watertight container 94, and a second end free (free end) in the vicinity of the anchor 3. Note that the length of the sensor cable 91 can be varied appropriately according to the depth for which ocean data needs to be measured, and does not necessarily have to extend to the sea bottom 2.

The electric watertight container 94 accommodates equipment such as a power source for supplying electric power to the sensors 95, a laser light source for injecting pulsed light into the optical fiber cable, and a photoelectric transducer for converting a light signal into an electric signal, for example. The equipment accommodated in the electric watertight container 94 is appropriately varied depending on the configuration of the sensor cable 91. One end of the sensor cable 91 is connected to the inside of the electric watertight container 94, to be electrically or optically connect to the equipment accommodated in the electric watertight container 94.

By placing the electric watertight container 94 in this manner, equipment of the sensor cable 91 can be placed in a watertight state in the sea, without installing the equipment in the observation buoy 8. Also, since the electric watertight container 94 is provided separately from the intermediate buoy 5, the electric watertight container 94 can be placed in the sea by use of the existing intermediate buoy 5.

Multiple restriction units 92 are placed on the submersible mooring cable 4, each of the restriction unit 92 being configured of a ring member having an opening through which the sensor cable 91 is insertable, for example. As shown in FIG. 1, the restriction unit 92 is spaced apart in the longitudinal direction of the submersible mooring cable 4. By arranging the restriction unit 92 in this manner, the sensor cable 91 can be kept from separating from the submersible mooring cable 4, merely by inserting the sensor cable 91 into the openings. Note that the restriction unit 92 is not limited to the configuration shown in FIG. 1, and may be configured of a tether connected to the submersible mooring cable 4, and an annular insertion part formed on the tip end of the tether.

Additionally, since the sensor cable 91 is placed such that it is longitudinally movable relative to the submersible mooring cable 4, the difference in stretch amount between the submersible mooring cable 4 and the sensor cable 91 due to difference in material can be tolerated. Note that although the lower end of the sensor cable 91 is a free end, the sensor cable 91 can be kept from coming off the restriction unit 92, since the ocean current C has an effect of locking the sensor cable 91 onto the restriction unit 92.

The signal cable 93 is, for example, an electric cable or an optical fiber cable, is placed along the intermediate mooring cable 6, and has a first end connected to the electric watertight container 94 and a second end connected to the observation buoy 8 as shown in FIG. 1. As shown in FIG. 2, the signal cable 93 is accommodated in the intermediate mooring cable 6 in a longitudinally movable manner, and is drawn to the outside from an intermediate part of the intermediate mooring cable 6, to be connected to the electric watertight container 94 with a certain slack in between, for example.

In an embodiment, the signal cable 93 may be accommodated in the intermediate mooring cable 6 by being wrapped by and interweaved into the intermediate mooring cable 6. With this configuration, even when a tensile force is applied to the intermediate mooring cable 6 by the ocean current C, the tensile force can be borne by the intermediate mooring cable 6, whereby tension on the signal cable 93 can be reduced to suppress damage in the signal cable 93.

Note that although not illustrated, the signal cable 93 may be placed along the intermediate mooring cable 6, by inserting the signal cable 93 into multiple ring members placed along the longitudinal direction of the intermediate mooring cable 6. Also, multiple ring members may be placed along the longitudinal direction of the signal cable 93, and the intermediate mooring cable 6 may be inserted into the ring members.

A first end of the signal cable 93 is electrically or optically connected to the equipment accommodated in the electric watertight container 94, and can transmit lower layer-ocean data acquired by the sensor cable 91 to the observation buoy 8. A second end of the signal cable 93 is connected to the storage unit 12 placed inside the observation buoy 8, and the lower layer-ocean data acquired by the sensor cable 91 is stored in the storage unit 12. Note that if the signal cable 93 is an optical fiber cable, the signal cable 93 may be connected to the storage unit 12 through a photoelectric transducer.

As has been described, by placing the lower layer-observation unit 9 along the submersible mooring cable 4, ocean data of the lower layer in locations deeper than the intermediate buoy 5 can be measured. In particular, by extending the sensor cable 91 close to the sea bottom 2, ocean data can be acquired over a wide area from the sea bottom 2 to the intermediate buoy 5.

Additionally, since ocean data of the lower layer is transmitted to the observation buoy 8 by the signal cable 93, the ocean data of the lower layer can be stored in the storage unit 12 placed in the observation buoy 8. Hence, the observation buoy 8 can accumulate upper layer-ocean data and lower layer-ocean data. By lifting the observation buoy 8 up to the sea surface 7 and communicating, ocean data of a wider area than in conventional techniques can be collectively transmitted to the user U in a ground base or on an observation ship.

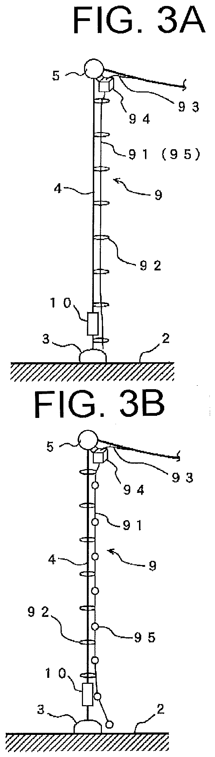

Hereinafter, modifications of the above-mentioned lower layer-observation unit 9 will be described with reference to FIGS. 3A to 3C. In a first modification of the lower layer-observation unit 9 shown in FIG. 3A, a sensor cable 91 is configured of an optical fiber sensor placed along the submersible mooring cable 4. By thus adopting an optical fiber sensor as the sensor cable 91, the sensor cable 91 (optical fiber sensor) itself can function as a sensor 95.

Pulsed light is injected into the optical fiber sensor, i.e., the sensor cable 91, from a laser light source accommodated in an electric watertight container 94. The incident light travels inside the optical fiber sensor while scattering, and a part of the scattered light returns to the inlet end as rear scattered light. Since Raman-scattered light, which is a type of scattered light, is temperature dependent, the temperature can be calculated by detecting the Raman-scattered light with a photodetector accommodated in the electric watertight container 94.

Also, occurrence of scattered light can be located, by measuring the time from the injection of pulsed light into the optical fiber sensor until the return of generated Raman-scattered light to the inlet end. Hence, by adopting an optical fiber sensor as the sensor cable 91, water temperature of the lower layer in locations deeper than the intermediate buoy 5 can be measured according to the depth.

In a second modification of the lower layer-observation unit 9 shown in FIG. 3B, a restriction unit 92 is connected to a sensor cable 91. In an embodiment, ring members that form the restriction unit 92 may be connected to the sensor cable 91, and a submersible mooring cable 4 may be inserted into openings of the ring members. This configuration can also keep the sensor cable 91 from separating from the submersible mooring cable 4, merely by inserting the submersible mooring cable 4 into the openings.

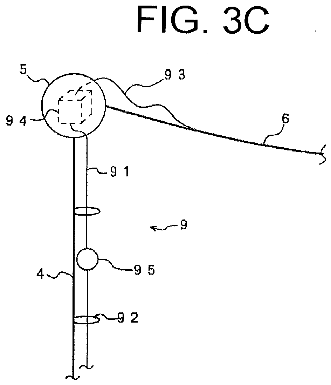

In a third modification of the lower layer-observation unit 9 shown in FIG. 3C, an electric watertight container 94 is accommodated in an intermediate buoy 5. This configuration can reduce the number of objects floating in the sea, and thereby reduce the influence of an ocean current C on an ocean data measurement system 1. In addition, if the intermediate buoy 5 is made watertight, the sealing performance of the electric watertight container 94 can be lowered or omitted. Also, the electric watertight container 94 may also serve as the intermediate buoy 5.

According to the ocean data measurement system 1 of the embodiment described above, ocean data of the upper layer from the sea surface 7 to the intermediate buoy 5 can be measured by the observation buoy 8, while ocean data of the lower layer in locations deeper than the intermediate buoy 5 can be measured by the lower layer-observation unit 9. Hence, ocean data can be acquired for a wider area than in conventional systems. Additionally, since the observation buoy 8 need not be sunk deeper than the intermediate buoy 5, the ocean data measurement system 1 acquiring ocean data of a wide area can be constructed at low cost.

Although an embodiment of the present invention has been described with reference to the accompanying drawings, the present invention is not limited to the above embodiments as a matter of course, and various modifications and amendments within the scope of claims naturally belong to the technical scope of the present invention.

A first aspect of the present invention includes: an anchor placed on the sea bottom; a submersible mooring cable having a first end connected to the anchor; an intermediate buoy connected to a second end of the submersible mooring cable and floating in the sea; an intermediate mooring cable having a first end connected to the intermediate buoy; an observation buoy connected to a second end of the intermediate mooring cable, and measuring ocean data of an upper layer from a sea surface to the intermediate buoy while floating and sinking between the intermediate buoy and the sea surface; and a lower layer-observation unit placed along the submersible mooring cable, and measuring ocean data of a lower layer in a location deeper than the intermediate buoy.

In the first aspect of the present invention, ocean data of the upper layer from the sea surface to the intermediate buoy is measured by the observation buoy, while ocean data of the lower layer in a location deeper than the intermediate buoy is measured by the lower layer-observation unit. Hence, ocean data of a wider area between the sea surface and the sea bottom can be acquired easily.

In a second aspect of the present invention, the lower layer-observation unit includes a sensor cable placed along the submersible mooring cable, a restriction unit keeping the sensor cable from separating from the submersible mooring cable, and a signal cable transmitting the measured ocean data of the lower layer to the observation buoy.

In the second aspect of the present invention, if a storage unit is placed in the observation buoy, for example, not only ocean data of the upper layer but also ocean data of the lower layer can be accumulated. By lifting the observation buoy up to the sea surface and communicating, ocean data of a wide area can be collectively transmitted to a ground base or an observation ship.

In a third aspect of the present disclosure, a plurality of the restriction units are placed on one of the submersible mooring cable and the sensor cable, each of the restriction unit being a ring member having an opening through which the other of the submersible mooring cable and the sensor cable is insertable.

In the third aspect of the present disclosure, if multiple restriction units, i.e., ring members are placed on the submersible mooring cable, for example, the sensor cable can be kept from separating from the submersible mooring cable, merely by inserting the sensor cable into the openings.

In a fourth aspect of the present invention, the lower layer-observation unit has an electric watertight container placed between the sensor cable and the signal cable.

In the fourth aspect of the present invention, equipment of the sensor cable can be placed in a watertight state in the sea, without installing the equipment in the observation buoy.

In a fifth aspect of the present invention, the signal cable is interweaved into the intermediate mooring cable.

In the fifth aspect of the present invention, even when a tensile force is applied to the intermediate mooring cable by an ocean current, the tensile force can be borne by the intermediate mooring cable, whereby tension on the signal cable can be reduced to suppress damage in the signal cable.

In a sixth aspect of the present invention, the observation buoy has a storage unit capable of storing ocean data of the upper layer and ocean data of the lower layer.

In the sixth aspect of the present invention, ocean data of a wide area from the sea surface to the sea bottom can be collectively stored in a concentrated manner, for example.

### Explanation of Reference Signs

- 1: ocean data measurement system
- 2: sea bottom
- 3: anchor
- 4: submersible mooring cable
- 5: intermediate buoy
- 6: intermediate mooring cable
- 61: ring portion
- 7: sea surface
- 8: observation buoy
- 81: pressure-resistant shell
- 82: through-hole
- 83: cover
- 9: lower layer-observation unit
- 10: acoustic release device
- 11: measuring portion
- 12: storage unit
- 13: specific gravity-adjustment mechanism
- 13a: buoyancy bag
- 13b: oil tank
- 13c: oil pump
- 14: antenna
- 91: sensor cable
- 92: restriction unit
- 93: signal cable
- 94: electric watertight container
- 95: sensor

## Claims

1. An ocean data measurement system comprising:
an anchor placed on a sea bottom;
a submersible mooring cable having a first end connected to the anchor;
an intermediate buoy connected to a second end of the submersible mooring cable and floating in the sea;
an intermediate mooring cable having a first end connected to the intermediate buoy;
an observation buoy connected to a second end of the intermediate mooring cable, and measuring ocean data of an upper layer from a sea surface to the intermediate buoy while floating and sinking between the intermediate buoy and the sea surface; and
a lower layer-observation unit placed along the submersible mooring cable, and measuring ocean data of a lower layer in a location deeper than the intermediate buoy.

2. The ocean data measurement system according to claim 1, wherein
the lower layer-observation unit includes
a sensor cable placed along the submersible mooring cable,
a restriction unit keeping the sensor cable from separating from the submersible mooring cable, and
a signal cable transmitting the measured ocean data of the lower layer to the observation buoy.

3. The ocean data measurement system according to claim 2, wherein
a plurality of the restriction units are placed on one of the submersible mooring cable and the sensor cable, each of the restriction unit being a ring member having an opening through which the other of the submersible mooring cable and the sensor cable is insertable.

4. The ocean data measurement system according to claim 2, wherein
the lower layer-observation unit has an electric watertight container placed between the sensor cable and the signal cable.

5. The ocean data measurement system according to claim 2, wherein
the signal cable is interweaved into the intermediate mooring cable.

6. The ocean data measurement system according to claim 1, wherein
the observation buoy has a storage unit capable of storing the ocean data of the upper layer and the ocean data of the lower layer.
